(19) Europäisches Patentamt
European Patent Office
Office européen des brevets

(11) Veröffentlichungsnummer: **0 358 081 A2**

(12) # EUROPÄISCHE PATENTANMELDUNG

(21) Anmeldenummer: **89115870.1**

(22) Anmeldetag: **29.08.89**

(51) Int. Cl.5: **C07D 239/42**

(30) Priorität: **02.09.88 DE 3829850**

(43) Veröffentlichungstag der Anmeldung: **14.03.90 Patentblatt 90/11**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Paust, Joachim, Dr.**
**Ringstrasse 3**
**D-6708 Neuhofen(DE)**
Erfinder: **Ernst, Hansgeorg, Dr.**
**Bussardweg 62**
**D-6720 Speyer(DE)**

(54) **Verfahren zur Herstellung von 2-n-Propyl-4-amino-5-methoxymethyl-pyrimidin.**

(57) Verbessertes Verfahren zur Herstellung von 2-n-propyl-4-aminosmethoxy methylpyrimidin der Formel I

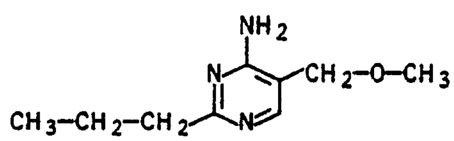

I

durch Umsetzung von Butyramidin II

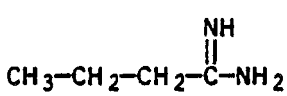

II

mit α-Methoxymethyl-β-methoxy-acrylnitril III,

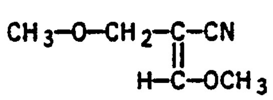

III

indem man das Butyramidin 11 mit einem 0,4 bis 5-molaren Überschuß von α-Methoxymethyl-β-methoxy-acrylnitril III bei Temperaturen von -10 bis +20 °C umsetzt.

EP 0 358 081 A2

## Verfahren zur Herstellung von 2-n-Propyl-4-amino-5-methoxymethyl-pyrimidin

Die vorliegende Erfindung betrifft ein verbessertes Verfahren zur Herstellung von 2-n-Propyl-4-amino-5-methoxymethyl-pyrimidin der Formel I

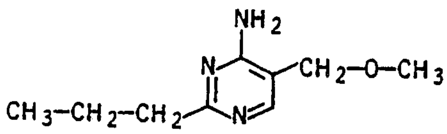

I

durch Umsetzung von Butyramidin 11

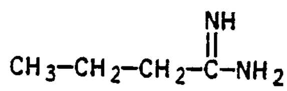

II

mit $\alpha$-Methoxymethyl-$\beta$-methoxy-acrylnitril III

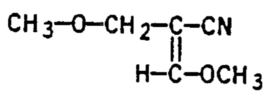

III

2-n-Propyl-4-amino-5-methoxymethyl-pyrimidin I ist das unmittelbare Vorprodukt für das in der Tiermedizin verwendete Coccidiostatikum Amprolium, welches aus I beispielsweise gemäß FR-PS 1 276 663 hergestellt werden kann.

Die Herstellung von I ist in den Patentschriften FR-PS 1 276 663 und GB-PS 953 876 beschrieben. Nach deren nahezu inhaltsgleichen Lehren wird das in situ aus Butyramidin-Hydrochlorid mit Natriummethylat freigesetzte Butyramidin II mit äquimolaren Mengen von $\alpha$-Methoxymethyl-$\beta$-methoxyacrylnitril in siedendem Methanol zu I umgesetzt. Die Isolierung des Wertprodukts I aus dem Reaktionsgemisch erfordert folgende Maßnahmen:

- Abdestillieren von Methanol
- Behandlung des Rückstands mit 50 %iger wäßriger Natronlauge in der Wärme
- Extraktion dieser Mischung mit Chloroform
- Eindampfen der Chloroformlösung
- Lösen des Rückstands in Xylol
- Einengen der Xylollösung
- Kristallisation unter Kühlung

Laut der GB-PS 953 876 wird nach diesem Verfahren eine nur mäßige Ausbeute an I in Höhe von 75, 1 % erzielt. In der französischen Patentschrift fehlen Angaben zur Ausbeute an I. Weder in der französischen noch in der britischen Patentschrift sind die physikalischen Daten des Produkts I oder Angaben über dessen Reinheit enthalten.

Abgesehen vom Nachteil einer mäßigen Ausbeute, erfordern die beschriebenen Verfahren aufgrund der vielstufigen Aufarbeitungsweise einen hohen Aufwand an Apparaten und Energie, und die Verwendung von dreierlei Lösungsmitteln - Methanol, Chloroform und Xylol - verursacht weitere Kosten.

Es bestand deshalb die Aufgabe, ein Verfahren zu finden, das die Herstellung von I in guten Ausbeuten und in hoher Reinheit, kostengünstig und ohne die Nachteile der bekannten Verfahren zu haben, ermöglicht.

Dementsprechend wurde ein verfahren zur Herstellung von 2-n-Propyl-4-amino-5-methoxymethylpyrimidin der Formel I

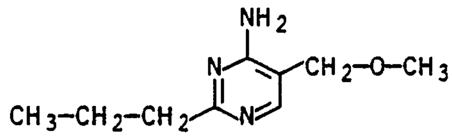

I

durch Umsetzung von Butyramidin II

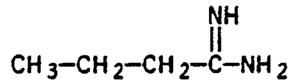

II

mit α-methoxymethyl-β-methoxy-acrylnitril III gefunden,

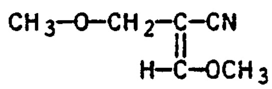

III

das dadurch gekennzeichnet ist, daß man das Butyramidin II mit einem 0,4 bis 5 molaren Überschuß von α-Methoxymethyl-β-methoxy-acrylnitril III bei Temperaturen von -10 bis +20° C umsetzt.

Die Umsetzung des Butyramidins II mit α-Methoxymethyl-β-methoxyacrylnitril erfolgt gemäß Reaktionsgleichung (1),

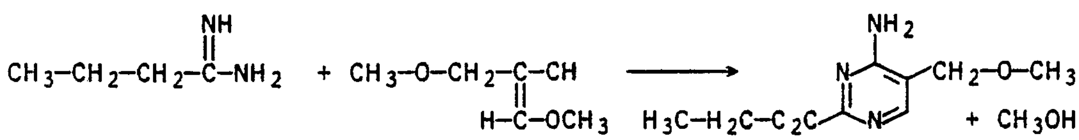

(1)

II III I

wobei pro Mol umgesetztem III ein Mol Methanol entsteht.

Der molare Überschuß von Verbindung III über das Butyramidin II beträgt vorteilhaft 0,4 bis 1,5 mol, vorzugsweise 0,8 bis 1,2 mol. Die Anwendung eines höheren Überschusses von III, beispielsweise eines 5-molaren Überschusses, ist möglich, führt aber zu keiner nennenswerten Verbesserung des Verfahrensergebnisses. Die Reaktion wird bei niedrigen Temperaturen, im allgemeinen bei -10 bis +20° C, vorteilhaft bei -5 bis 15° C und insbesondere bei 0 bis 10° C ausgeführt.

Beim erfindungsgemäßen Verfahren wird die Bildung unerwünschter und weder durch Destillation noch durch Kristallisation abtrennbarer Nebenprodukte unterdrückt. Das Pyrimidin 1 entsteht deshalb in hoher Reinheit > 99 % und kann ohne weitere Aufarbeitung aus dem Reaktionsgemisch isoliert werden, beispielsweise durch Kristallisation oder Destillation. Die beim erfindungsgemäßen Verfahren erzielbare Ausbeute beträgt über 90 % d.Th.

Bei der Durchführung des Verfahrens kann auf verschiedene Art und Weise vorgegangen werden und die Wahl, welche der im folgenden genannten möglichen Verfahrensweisen angewandt wird, richtet sich im allgemeinen nach den betrieblichen Gegebenheiten, beispielsweise nach der Verfügbarkeit entsprechender apparativer Einrichtungen.

Soll das Wertprodukt I durch Kristallisation aus dem Reaktionsgemisch isoliert werden, so ist es zweckmäßig, das Butyramidin II in einer Form zu verwenden, die weitgehend oder vorteilhaft völlig frei von salzartigen Begleitstoffen ist.

Butyramidin solcher Qualität kann beispielsweise in einer separaten Stufe aus Butyramidin-Hydrochlorid - der Handels- und Lagerform des Butyramidins - durch Umsetzung mit einer starken, nichtwäßrigen Base erzeugt werden, wobei die entsprechenden Salze als unlösliche Niederschläge anfallen. Die Base kann in stöchiometrischen Mengen als auch in geringem Unterschuß verwendet werden. Als Base wird aufgrund seiner Preiswürdigkeit zweckmäßigerweise Natriummethanolat in Form seiner handelsüblichen, 30 %igen methanolischen Lösung eingesetzt. Es ist aber auch möglich, andere Basen wie Natriumethanolat, Kalium-tert.butanolat oder Kaliumamid zu verwenden. Ebenso kann das Methanol durch andere Lösungsmittel oder

durch Lösungsmittelgemische ersetzt werden. Vorteilhaft wählt man zu diesem zweck solche Lösungsmittel, die sich unter den Reaktionsbedingungen zur Herstellung des Pyrimidins I inert verhalten, wie Ethanol, Isopropanol, Tetrahydrofuran oder Dioxan; andernfalls sollten die Lösungsmittel vor dem Einsatz des Butyramidins im erfindungsgemäßen Verfahren weitgehend abgetrennt werden.

Die bei der Herstellung des freien Butyramidins II erforderliche Lösungsmittelmenge wird von der gewünschten Viskosität der Butyramidinlösung bestimmt, und diese richtet sich wiederum nach den jeweiligen Apparaten und Verfahren, beispielsweise Filtration oder Zentrifugation, mit bzw. nach denen die in der Butyramidinlösung suspendierten Salze abgetrennt werden.

Auf diese Weise erhaltene Butyramidinlösungen werden vor ihrem Einsatz in die Reaktion zweckmäßigerweise aufkonzentriert, so daß ihr Lösungsmittelgehalt im allgemeinen zwischen 10 und 20 Gew.% beträgt. Üblicherweise führt man die Konzentrierung bei vermindertem Druck und Temperaturen von 20 bis 40 °C durch. Es ist auch möglich, das Lösungsmittel vollständig aus den Butyramidinlösungen zu entfernen, doch führt dies im allgemeinen zu keinen weiteren Vorteilen.

Das derart gewonnene Butyramidin II wird in die Reaktion eingesetzt. Dabei dosiert man das Butyramidin vorteilhaft zu dem im Reaktionsgefäß vorgelegten α-Methoxymethyl-β-methoxy-acrylnitril III. Die Durchführung der Reaktion in Gegenwart eines zusätzlichen Lösungsmittels ist möglich, bietet in der Regel aber keine weiteren Vorteile, da das im Reaktionsgefäß vorhandene, überschüssige III und das im Zuge der Reaktion gebildete Methanol als Lösungsmittel fungieren und die Rührbarkeit der Reaktionsmischung gewährleisten.

Bereits während der Reaktion scheidet sich das gebildete Pyrimidin I ab und kann nach beendeter Reaktion, beispielsweise durch Filtration oder Zentrifugation, von der Mutterlauge abgetrennt werden. Das Wertprodukt I fällt dabei in so hoher Reinheit an, daß es praktisch nur noch mit einem geeigneten Lösungsmittel, beispielsweise Methyl-tert.-butylether (MTB), nachgewaschen werden muß, um anhaftendes III zu entfernen.

Als besonders vorteilhafte Verfahrensweise stellte sich heraus, die mit der Waschflüssigkeit vereinigte Mutterlauge als Lösungsmittel für weitere Umsetzungen gemäß Reaktion (1) zu verwenden.

Dazu wird die Mutterlauge, die neben dem überschüssigen Ausgangsmaterial III noch erhebliche Mengen des Produkts I enthält, im allgemeinen bei Temperaturen von 20 bis 30 °C und bei vermindertem Druck weitgehend von Methanol und MTB befreit. Man ergänzt dann das verbrauchte α-Methoxy-methyl-β-methoxy-acrylnitril III und fügt dieser Mischung dann wieder, wie beschrieben, das Butyramidin II zu und zwar, wie beschrieben, in solchen Mengen, daß der Überschuß an III über II 0,4 bis 1,5 molar ist. Die Reaktion wird dann, wie beschrieben, bei Temperaturen von -10 bis 20 °C durchgeführt, wonach das entstandene Produkt 1 auf analoge Weise abgetrennt wird. Dieser Vorgang kann praktisch beliebig oft wiederholt werden.

Zieht man es vor, das Pyrimidin I destillativ aus dem Reaktionsgemisch zu isolieren, so kann die Umsetzung - im Hinblick auf die Reaktionsführung und die Verwendungsform des Butyramidins - wie oben beschrieben ausgeführt werden. Es kann aber auch zweckmäßig sein, das Butyramidin II aus seinem Hydrochlorid in situ, d.h. in Gegenwart von vorgelegtem III im Reaktionsgefäß, freizusetzen, wobei die gleichen Basen angewendet werden können, wie bei der separaten Freisetzung des Butyramidins II.

Vorteilhaft geht man dabei so vor, daß man das Butyramidin-Hydrochlorid in überschüssigen III vorlegt und zu dieser Suspension, im Temperaturbereich von -10 bis 20 °C, eine Lösung der Base, beispielsweise 30 %ige methanolische Natriummethanolatlösung tropft. Die im Zuge der Butyramidinfreisetzung abgeschiedenen Salze werden zweckmäßigerweise nach beendeter Umsetzung und vor der Destillation der Reaktionsmischung auf übliche Art und Weise abgetrennt. Es versteht sich von selbst, daß man bei dieser Ausführungsform soviel Lösungsmittel zum Reaktionsgemisch gibt, damit das im Zuge der Reaktion gebildete Pyrimidin I nicht auskristallisiert, da andernfalls das Wertprodukt I mitsamt der Salze abgetrennt würde.

Die Destillation der Reaktionsmischung wird auf die übliche Weise durchgeführt, indem man zunächst das Lösungsmittel, gegebenenfalls unter vermindertem Druck, destillativ entfernt und dann den zurückbleibenden, schwerflüchtigen Rückstand, der neben dem Produkt 1 noch die überschüssige Ausgangsverbindung III enthält, bei vermindertem Druck fraktionierend destilliert.

An die Reaktionsgefäße werden beim erfindungsgemäßen Verfahren keine besonderen Anforderungen gestellt. Im allgemeinen verwendet man einfache Rührkessel, welche mit einer Kühlvorrichtung versehen sind.

Die verwendeten Ausgangsmaterialien sind ebenso wie die Methoden zu ihrer Herstellung bekannt (s. z.B. FR-PS 1 276 663) oder können käuflich erworben werden. α-Methoxymethyl-β-methoxy-acrylnitril kann als Mischung seiner E,Z-Isomeren verwendet werden.

## Beispiele

### Beispiel 1

12,96 g (0,105 mol) Butyramidin-Hydrochlorid wurden in 50 ml Methanol suspendiert und bei 2 bis 5° C mit 18, 0 g einer 30 %igen Lösung von Natriummethanolat (0, 10 mol) in Methanol versetzt. Das ausgefallene Natriumchlorid wurde abfiltriert und mit 5 ml Methanol nachgewaschen. Das Filtrat wurde am Rotationsverdampfer bei 30° C Badtemperatur und bei einem Druck von 5 mbar eingeengt.

Das so erhaltene rohe Butyramidin II (Methanolgehalt ca. 10 Gew.%) wurde innerhalb von 15 min bei 2 bis 5° C zu 25,4 g (0,2 mol) E,Z-α-Methoxy-methyl-β-methoxy-acrylnitril III getropft. Es wurde 6 h bei 5° C gerührt, wobei ein Teil des gewünschten Produkts I auskristallisierte. Das kristalline Produkt wurde unter Anwendung von Unterdruck abfiltriert und die Kristalle mit wenig MTB gewaschen. Ausbeute: 8,88 g (38 % d.Th.).

Die Mutterlauge wurde bei 30° C Badtemperatur und einem Druck von 5 mbar weitgehend von Methanol und MTB befreit und nach Ergänzung mit 12,7 g (0,1 mol) III, wie oben beschrieben, mit 0,1 mol II versetzt. Es wurde 10 h bei 2 bis 5° C gerührt. Die abgeschiedenen Kristalle wurden wie beschrieben isoliert.
Ausbeute: 15,85 g (87,6 % d.Th.) Schmelzpunkt: 67-70° C.

5 weitere Umsetzungen in der oben beschriebenen Weise, d.h. unter Wiederverwendung der Mutterlauge, lieferten eine durchschnittliche Ausbeute an I in Höhe von 91,6 % d.Th.

### Beispiel 2

12,35 g (0,1 mol) Butyramidin-Hydrochlorid wurden in 27,23 g (0,2 mol) III suspendiert und bei 2 bis 5° C, innerhalb von 15 min, tropfenweise mit 18 g einer 30 %igen Lösung von Natriummethanolat (0, 1 mol) in Methanol versetzt. Es wurde 10 h bei 10° C gerührt und anschließend das abgeschiedene Natriumchlorid abfiltriert. Das Methanol wurde am Rotationsverdampfer abdestilliert. Der Rückstand wurde fraktionierend unter vermindertem Druck destilliert. Dabei wurden 11,56 g (0,091 mol) III, Kp. 75-81° C, 0,5 mbar und 16,11 g (89 % d.Th.) I, Kp. 104-107° C, 0,4 mar, erhalten.

## Ansprüche

1. Verfahren zur Herstellung von 2-n-Propyl-4-amino-5-methoxy-methylpyrimidin der Formel I

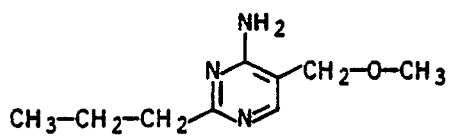

I

durch Umsetzung von Butyramidin II

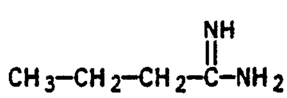

II

mit α-Methoxymethyl-β-methoxy-acrylnitril III

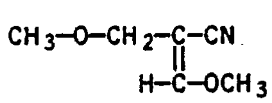

III

dadurch gekennzeichnet, daß man das Butyramidin II mit einem 0,4 bis 5 molaren Überschuß von α-Methoxymethyl-β-methoxy-acrylnitril III bei Temperaturen von -10 bis +20 °C umsetzt.

2. Verfahren nach Anspruch 1, dadurch gekennzeichnet, daß man das Butyramidin II in einer Form einsetzt, die weitgehend frei ist von salzartigen Begleitstoffen.

3. Verfahren nach Anspruch 1, dadurch gekennzeichnet, daß man das Butyramidin II in einer Form einsetzt, die weitgehend frei ist von salzartigen Begleitstoffen, und daß man das aus seiner Umsetzung mit α-Methoxymethyl-β-methoxy-acrylnitril erhaltene Produkt I aus der Reaktionsmischung auskristallisiert, abtrennt und die Mutterlauge als Lösungsmittel für weitere Umsetzungen von II mit III verwendet.

4. Verfahren nach Anspruch 1, dadurch gekennzeichnet, daß man das Produkt 2-n-Propyl-4-amino-5-methoxymethylpyrimidin I destillativ aus dem Reaktionsgemisch isoliert.